# EUROPEAN PATENT SPECIFICATION

(11) **EP 4 040 999 B1**
(45) Date of publication and mention of the grant of the patent: **16.09.2026**
(21) Application number: 20793080.1
(22) Date of filing: 08.10.2020
(51) Int. Cl.: A24B 15/167, A24B 15/30, A24F 40/42, A61K 31/352

(54) **AEROSOLISABLE MATERIAL**
AEROSOLFÄHIGES MATERIAL
MATÉRIAU AÉROSOLISABLE

(30) Priority: 09.10.2019 US 201962912975 P
(43) Date of publication of application: 17.08.2022
(73) Proprietor: Nicoventures Trading Limited, London WC2R 3LA (GB); RAI Strategic Holdings, Inc., Winston-Salem NC 27101 (US)
(72) Inventor: DAVIS, Michael Foster, Winston-Salem, North Carolina 27101 (US); CARAWAY, John Will, Winston-Salem, North Carolina 27101 (US); MONTSERRAT SANCHEZ PENA, Maria, London WC2R 3LA (GB)
(74) Representative: D Young & Co LLP
(86) International application number: PCT/GB2020/052503
(87) International publication number: WO 2021/069908

(56) References cited:
- GB-A- 2 569 961
- US-A1- 2015 181 924
- US-A1- 2018 289 062
- US-A1- 2018 325 164

## Description

### Field

The present disclosure relates to an aerosolisable material, a method of making said material, as well as containers and systems comprising and using said material.

### Background

Aerosol delivery systems which generate an aerosol for inhalation by a user are known in the art. Such systems typically comprise an aerosol generator which is capable of converting an aerosolisable material into an aerosol. In some instances, the aerosol generated is a condensation aerosol whereby an aerosolisable material is heated to form a vapor which is then allowed to condense into an aerosol. In other instances, the aerosol generated is an aerosol which results from the atomization of the aerosolisable material. Such atomization may be brought about mechanically, e.g. by subjecting the aerosolisable material to vibrations so as to form small particles of material that are entrained in airflow. Alternatively, such atomization may be brought about electrostatically, or in other ways, such as by using pressure etc.

Depending on the constituents of the aerosolisable material that are to be provided to a user, it may be preferable to formulate the aerosolisable material in a certain way. For example, it may be preferable to formulate the aerosolisable material so as to produce an aerosol with a particular profile. It may also be preferable to formulate the aerosolisable material so as to ensure the aerosolisable material meets certain standards of quality, consistency and the like.

It would thus be desirable to provide an aerosolisable material that is formulated so as to be acceptable to a user.

Documents US2015/181924A1, US2018/325164A1, GB2569961A, US2018/289062A1 disclose aerosolisable formulations comprising cannabidiol.

### Summary

In one aspect there is provided an aerosolisable material comprising (+)-cannabidiol and at least one carrier constituent.

In a further aspect there is provided an article comprising the aerosolisable material as defined herein.

In a further aspect there is provided an aerosol provision system comprising an aerosol provision device and an article as defined herein.

In a further aspect there is provided a method for producing the aerosolisable material as defined herein, the method comprising combining (+)-cannabidiol with at least one carrier constituent.

These aspects and other aspects will be apparent from the following detailed description. In this regard, particular sections of the description are not to be read in isolation from other sections.

### Brief Description of the Drawings

Various embodiments will now be described in detail by way of example only with reference to the accompanying drawings in which:

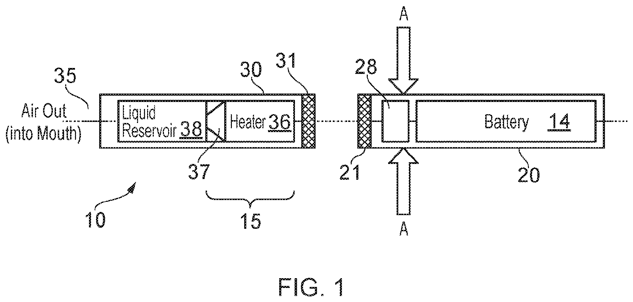

Figure 1 - Provides a schematic overview of an article, aerosol delivery device and system as described herein.

### Detailed Description

In one aspect there is provided an aerosolisable material comprising (+)-cannabidiol and at least one carrier constituent.

Naturally occurring cannabidiol is present as (-)-cannabidiol. For example, cannabidiol extracted from natural sources, such as from Cannabis sativa, is present as (-)-cannabidiol.

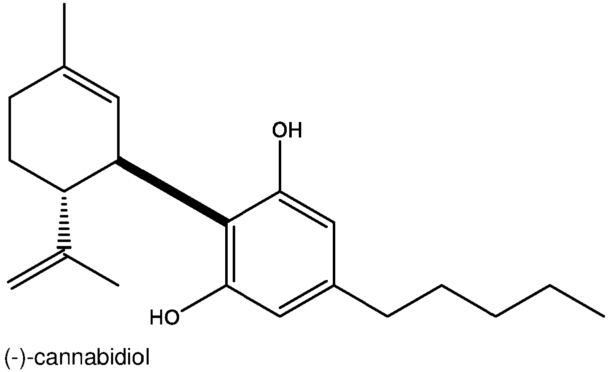

It is, however, possible to prepare synthetic forms of cannabidiol, including the (+)-enantiomer, (+)-cannabidiol.

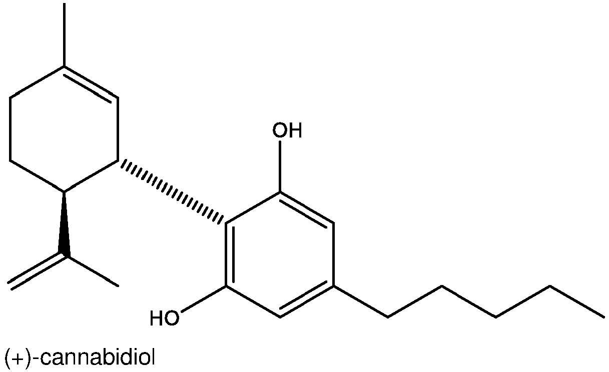

In this regard, reference can be made to the Shah, V.J., Synthesis of cannabidiol stereoisomers and analogs as potential anticonvulsant agents. The University of Arizona.

Cannabinoid receptors are of a class of cell membrane receptors in the G protein-coupled receptor superfamily.

There are currently two known subtypes of cannabinoid receptors, termed CB1 and CB2. The CB1 receptor is thought to be expressed mainly in the brain (central nervous system or "CNS"), but also in the lungs, liver and kidneys. The CB2 receptor is thought to be expressed mainly in the immune system and in hematopoietic cells.

It has been found that (+)-cannabidiol displays increased selectivity for the CB2 receptor compared to the CB1 receptor. This is in contrast to (-)-cannabidiol, which generally does not show any selectivity between the two receptors. In this regard, see Br J Pharmacol. 2001 Oct; 134(4): 845-852.

In some embodiments, at least 1% of the cannabidiol present in the aerosolisable material is present in the form of (+)-cannabidiol. In some embodiments, at least 5% of the cannabidiol present in the aerosolisable material is present in the form of (+)-cannabidiol. In some embodiments, at least 10% of the cannabidiol present in the aerosolisable material is present in the form of (+)-cannabidiol. In some embodiments, at least 15% of the cannabidiol present in the aerosolisable material is present in the form of (+)-cannabidiol. In some embodiments, at least 20% of the cannabidiol present in the aerosolisable material is present in the form of (+)-cannabidiol. In some embodiments, at least 25% of the cannabidiol present in the aerosolisable material is present in the form of (+)-cannabidiol. In some embodiments, at least 30% of the cannabidiol present in the aerosolisable material is present in the form of (+)-cannabidiol. In some embodiments, at least 35% of the cannabidiol present in the aerosolisable material is present in the form of (+)-cannabidiol. In some embodiments, at least 40% of the cannabidiol present in the aerosolisable material is present in the form of (+)-cannabidiol. In some embodiments, at least 45% of the cannabidiol present in the aerosolisable material is present in the form of (+)-cannabidiol. In some embodiments, at least 50% of the cannabidiol present in the aerosolisable material is present in the form of (+)-cannabidiol. In some embodiments, at least 55% of the cannabidiol present in the aerosolisable material is present in the form of (+)-cannabidiol. In some embodiments, at least 60% of the cannabidiol present in the aerosolisable material is present in the form of (+)-cannabidiol. In some embodiments, at least 65% of the cannabidiol present in the aerosolisable material is present in the form of (+)-cannabidiol. In some embodiments, at least 70% of the cannabidiol present in the aerosolisable material is present in the form of (+)-cannabidiol. In some embodiments, at least 75% of the cannabidiol present in the aerosolisable material is present in the form of (+)-cannabidiol. In some embodiments, at least 80% of the cannabidiol present in the aerosolisable material is present in the form of (+)-cannabidiol. In some embodiments, at least 85% of the cannabidiol present in the aerosolisable material is present in the form of (+)-cannabidiol. In some embodiments, at least 90% of the cannabidiol present in the aerosolisable material is present in the form of (+)-cannabidiol. In some embodiments, at least 95% of the cannabidiol present in the aerosolisable material is present in the form of (+)-cannabidiol. In some embodiments, substantially all of the cannabidiol present in the aerosolisable material is present in the form of (+)-cannabidiol.

Where less than 100% of the cannabidiol is present in the form of (+)-cannabidiol, the remaining amount of cannabidiol can be present in the form of (-)-cannabidiol.

The cannabidiol may be present in the aerosolisable material based on a mg/ml basis of the aerosolisable material.

In one embodiment, the cannabidiol is present in an amount of from about 5 mg/ml up to about 100 mg/ml. In one embodiment, the cannabidiol is present in an amount of from about 5 mg/ml up to about 90 mg/ml. In one embodiment, the cannabidiol is present in an amount of from about 5 mg/ml up to about 80 mg/ml. In one embodiment, the cannabidiol is present in an amount of from about 5 mg/ml up to about 70 mg/ml. In one embodiment, the cannabidiol is present in an amount of from about 5 mg/ml up to about 60 mg/ml. In one embodiment, the cannabidiol is present in an amount of from about 5 mg/ml up to about 50 mg/ml. In one embodiment, the cannabidiol is present in an amount of from about 5 mg/ml up to about 40 mg/ml. In one embodiment, the cannabidiol is present in an amount of from about 5 mg/ml up to about 30 mg/ml. In one embodiment, the cannabidiol is present in an amount of from about 5 mg/ml up to about 20 mg/ml. In one embodiment, the cannabidiol is present in an amount of from about 5 mg/ml up to about 10 mg/ml.

In one embodiment, the cannabidiol is present in an amount of about 5 mg/ml or more. In one embodiment, the cannabidiol is present in an amount of about 10 mg/ml or more. In one embodiment, the cannabidiol is present in an amount of about 15 mg/ml or more. In one embodiment, the cannabidiol is present in an amount of about 20 mg/ml or more. In one embodiment, the cannabidiol is present in an amount of about 25 mg/ml or more. In one embodiment, the cannabidiol is present in an amount of about 30 mg/ml or more. In one embodiment, the cannabidiol is present in an amount of about 35 mg/ml or more. In one embodiment, the cannabidiol is present in an amount of about 40 mg/ml or more. In one embodiment, the cannabidiol is present in an amount of about 45 mg/ml or more. In one embodiment, the cannabidiol is present in an amount of about 50 mg/ml or more. In one embodiment, the cannabidiol is present in an amount of about 55 mg/ml or more. In one embodiment, the cannabidiol is present in an amount of about 60 mg/ml or more. In one embodiment, the cannabidiol is present in an amount of about 65 mg/ml or more.

The carrier constituent comprises one or more constituents capable of forming an aerosol, particularly when evaporated and allowed to condense. In some embodiments, the carrier constituent may comprise one or more of glycerol, propylene glycol, triethylene glycol, tetraethylene glycol, 1,3-butylene glycol, erythritol, meso-Erythritol, ethyl vanillate, ethyl laurate, a diethyl suberate, triethyl citrate, triethylene glycol diacetate, triacetin, a diacetin mixture, benzyl benzoate, benzyl phenyl acetate, tributyrin, lauryl acetate, lauric acid, myristic acid, and propylene carbonate.

In one embodiment, the carrier constituent comprises propylene glycol.

In one embodiment, propylene glycol is present in an amount of from 10%w/w to 95%w/w based on the total weight of the material. In one embodiment, propylene glycol is present in an amount of from 20%w/w to 95%w/w based on the total weight of the material. In one embodiment, propylene glycol is present in an amount of from 30%w/w to 95%w/w based on the total weight of the material. In one embodiment, propylene glycol is present in an amount of from 40%w/w to 95%w/w based on the total weight of the material.

In one embodiment, propylene glycol is present in an amount of from 50%w/w to 90%w/w based on the total weight of the material. In one embodiment, propylene glycol is present in an amount of from 50%w/w to 85%w/w based on the total weight of the material. In one embodiment, propylene glycol is present in an amount of from 50%w/w to 80%w/w based on the total weight of the material. In one embodiment, propylene glycol is present in an amount of from 50%w/w to 75%w/w based on the total weight of the material. In one embodiment, propylene glycol is present in an amount of from 50%w/w to 60%w/w based on the total weight of the material. In one embodiment, propylene glycol is present in an amount of from 50%w/w to 65%w/w based on the total weight of the material. In one embodiment, propylene glycol is present in an amount of from 50%w/w to 60%w/w based on the total weight of the material.

In one embodiment, propylene glycol is present in an amount of from 55%w/w to 90%w/w based on the total weight of the material. In one embodiment, propylene glycol is present in an amount of from 60%w/w to 90%w/w based on the total weight of the material. In one embodiment, propylene glycol is present in an amount of from 65%w/w to 90%w/w based on the total weight of the material. In one embodiment, propylene glycol is present in an amount of from 70%w/w to 90%w/w based on the total weight of the material. In one embodiment, propylene glycol is present in an amount of from 75%w/w to 90%w/w based on the total weight of the material. In one embodiment, propylene glycol is present in an amount of from 80%w/w to 90%w/w based on the total weight of the material. In one embodiment, propylene glycol is present in an amount of from 85%w/w to 90%w/w based on the total weight of the material.

In one embodiment, propylene glycol is present in an amount of at least 10%w/w based on the total weight of the material. In one embodiment, propylene glycol is present in an amount of at least 20%w/w based on the total weight of the material. In one embodiment, propylene glycol is present in an amount of at least 30%w/w based on the total weight of the material. In one embodiment, propylene glycol is present in an amount of at least 40%w/w based on the total weight of the material. In one embodiment, propylene glycol is present in an amount of at least 50%w/w based on the total weight of the material. In one embodiment, propylene glycol is present in an amount of at least 55%w/w based on the total weight of the material. In one embodiment, propylene glycol is present in an amount of at least 60%w/w based on the total weight of the material. In one embodiment, propylene glycol is present in an amount of at least 65%w/w based on the total weight of the material. In one embodiment, propylene glycol is present in an amount of at least 70%w/w based on the total weight of the material. In one embodiment, propylene glycol is present in an amount of at least 75%w/w based on the total weight of the material. In one embodiment, propylene glycol is present in an amount of at least 80%w/w based on the total weight of the material. In one embodiment, propylene glycol is present in an amount of at least 85%w/w based on the total weight of the material. In one embodiment, propylene glycol is present in an amount of at least 90%w/w based on the total weight of the material.

In one embodiment, propylene glycol is present in an amount of about 70%w/w.

In some embodiments, the w/w% amount of propylene glycol in the material, based on the total weight of the material, is equal to or above a threshold C_{%}, the threshold being defined according to ${C}_{%} = 11.416 \times {\left(A\right)}^{0.377}$ wherein A is the amount of the at least one cannabinoid present in the material in mg/ml. It has been found that aerosolisable materials comprising at least one cannabinoid, such as cannabidiol, and propylene glycol conforming to the above threshold, are particularly stable.

In some embodiments, the amount of propylene glycol in the system is above the threshold C_{%}. For example, the amount of propylene glycol may be about 1w/w%, 2w/w%, 3w/w%, 4w/w%, 5w/w%, 6w/w%, 7w/w%, 8w/w%, 9w/w% or 10w/w% above the threshold C_{%}. Including more propylene glycol relative to the threshold can be important if the aerosolisable material attracts water during storage. This additional propylene glycol can therefore prevent the CBD from precipitating during periods of storage.

In some embodiments, the aerosolisable material comprises less than 12%w/w water. In some embodiments, the aerosolisable material comprises less than 11%w/w water. In some embodiments, the aerosolisable material comprises less than 10%w/w water. In some embodiments, the aerosolisable material comprises less than 5%w/w water. In some embodiments, the aerosolisable material comprises less than 1%w/w water. In some embodiments, the aerosolisable material comprises less than 0.5%w/w water. In some embodiments, the aerosolisable material comprises substantially no water.

In one embodiment, the carrier constituent comprises glycerol.

In one embodiment, glycerol is present in an amount of from 10%w/w to 95%w/w based on the total weight of the material. In one embodiment, glycerol is present in an amount of from 20%w/w to 95%w/w based on the total weight of the material. In one embodiment, glycerol is present in an amount of from 30%w/w to 95%w/w based on the total weight of the material. In one embodiment, glycerol is present in an amount of from 40%w/w to 95%w/w based on the total weight of the material. In one embodiment, glycerol is present in an amount of from 50%w/w to 95%w/w based on the total weight of the material.

In one embodiment, glycerol is present in an amount of from 50%w/w to 90%w/w based on the total weight of the material. In one embodiment, glycerol is present in an amount of from 50%w/w to 85%w/w based on the total weight of the material. In one embodiment, glycerol is present in an amount of from 50%w/w to 80%w/w based on the total weight of the material. In one embodiment, glycerol is present in an amount of from 50%w/w to 75%w/w based on the total weight of the material. In one embodiment, glycerol is present in an amount of from 50%w/w to 60%w/w based on the total weight of the material. In one embodiment, glycerol is present in an amount of from 50%w/w to 65%w/w based on the total weight of the material. In one embodiment, glycerol is present in an amount of from 50%w/w to 60%w/w based on the total weight of the material.

In one embodiment, glycerol is present in an amount of from 55%w/w to 90%w/w based on the total weight of the material. In one embodiment, glycerol is present in an amount of from 60%w/w to 90%w/w based on the total weight of the material. In one embodiment, glycerol is present in an amount of from 65%w/w to 90%w/w based on the total weight of the material. In one embodiment, glycerol is present in an amount of from 70%w/w to 90%w/w based on the total weight of the material. In one embodiment, glycerol is present in an amount of from 75%w/w to 90%w/w based on the total weight of the material. In one embodiment, glycerol is present in an amount of from 80%w/w to 90%w/w based on the total weight of the material. In one embodiment, glycerol is present in an amount of from 85%w/w to 90%w/w based on the total weight of the material.

In one embodiment, glycerol is present in an amount of at least 10%w/w based on the total weight of the material. In one embodiment, glycerol is present in an amount of at least 20%w/w based on the total weight of the material. In one embodiment, glycerol is present in an amount of at least 30%w/w based on the total weight of the material. In one embodiment, glycerol is present in an amount of at least 40%w/w based on the total weight of the material. In one embodiment, glycerol is present in an amount of at least 50%w/w based on the total weight of the material. In one embodiment, glycerol is present in an amount of at least 50%w/w based on the total weight of the material. In one embodiment, glycerol is present in an amount of at least 55%w/w based on the total weight of the material. In one embodiment, glycerol is present in an amount of at least 60%w/w based on the total weight of the material. In one embodiment, glycerol is present in an amount of at least 65%w/w based on the total weight of the material. In one embodiment, glycerol is present in an amount of at least 70%w/w based on the total weight of the material. In one embodiment, glycerol is present in an amount of at least 75%w/w based on the total weight of the material. In one embodiment, glycerol is present in an amount of at least 80%w/w based on the total weight of the material. In one embodiment, glycerol is present in an amount of at least 85%w/w based on the total weight of the material. In one embodiment, glycerol is present in an amount of at least 90%w/w based on the total weight of the material.

In one embodiment, both glycerol and propylene glycol are present as carrier constituents.

In one embodiment, glycerol and propylene glycol are present in the aerosolisable material in the following amounts:
60 to 90%w/w propylene glycol; and
40 to 10%w/w glycerol,
based on the total weight of glycerol and propylene glycol present in the material.

In one embodiment, glycerol and propylene glycol are present in the aerosolisable material in the following amounts:
70 to 80%w/w propylene glycol; and
30 to 20%w/w glycerol.
based on the total weight of glycerol and propylene glycol present in the material.

In one embodiment, the aerosolisable material comprises about 70%w/w propylene glycol and about 30% glycerol.

At room temperature, some cannabinoids, such as cannabidiol, are crystalline solids. Therefore, in order to be provided in liquid form, it is generally required to provide a solution containing the cannabinoid. However, some cannabinoids, such as cannabidiol, are insoluble in water. On the other hand, water is often present in aerosolisable materials (liquids) that are used in aerosol provision systems such as e-cigarettes. The presence of such water could inhibit the solubility of the cannabinoid in the aerosolisable material, and such inhibition may be mitigated by additionally using a surface active constituent, such as an emulsifier or surfactant. However, it may not be desirable to include such surface active constituents due to user acceptability. For example, the inclusion of such surface active constituents may lead to an unacceptable flavor. The present inventors have found that it is possible to provide a stable aerosolisable material comprising at least one cannabinoid, such as cannabidiol, without the need for surface active constituents.

Therefore, in some embodiments, the aerosolisable material disclosed herein does not comprise a surface active constituent. Examples of surface active constituents include medium chain triglycerides (MCT) and tocopherol acetate.

In some embodiments, the aerosolisable material has a turbidity of about 10 NTU or less.

In this regard, the present inventors have found that when preparing an aerosolisable material comprising a cannabinoid, such as cannabidiol, it is desirable to ensure that the turbidity of the material is 10 NTU or less. When the turbidity of the material is above this range, it is a sign that one or more of the constituents of the material is not present in the material in a stable manner. This could impact the use of the aerosolisable material in a number of ways. For example, the user may perceive the lack of stability and form an opinion that the aerosolisable material is of inferior quality. Alternatively or additionally, such instability may lead to inefficient transfer of one or more constituents from the aerosolisable material to the aerosol. Likewise, such instability may lead to the aerosolisable material causing suboptimal performance of any system or device using the material. The present inventors have found that issues of stability may be particularly pronounced when the aerosolisable material comprises a cannabinoid and have thus found that ensuring the aerosolisable material has a turbidity of 10 NTU or less is important.

In one embodiment, the aerosolisable material is a liquid at about 25°C.

The aerosolisable material may comprise one or more further constituents. In particular, one or more further constituents may be selected from one or more physiologically and/or olfactory active constituents, and/or one or more functional constituents.

In some embodiments, the active constituent is a physiologically active constituent and may be selected from nicotine, nicotine salts (e.g. nicotine ditartrate/nicotine bitartrate), nicotine-free tobacco substitutes, other alkaloids such as caffeine, or mixtures thereof.

In some embodiments, the active constituent is an olfactory active constituent and may be selected from a "flavour" and/or "flavourant" which, where local regulations permit, may be used to create a desired taste, aroma or sensation in a product for adult consumers. In some instances such constituents may be referred to as flavours, flavourants, cooling agents, heating agents, or sweetening agents, and may include one or more of extracts (e.g., licorice, hydrangea, Japanese white bark magnolia leaf, chamomile, fenugreek, clove, menthol, Japanese mint, aniseed, cinnamon, herb, wintergreen, cherry, berry, peach, apple, Drambuie, bourbon, scotch, whiskey, spearmint, peppermint, lavender, cardamom, celery, cascarilla, nutmeg, sandalwood, bergamot, geranium, honey essence, rose oil, vanilla, lemon oil, orange oil, cassia, caraway, cognac, jasmine, ylang-ylang, sage, fennel, piment, ginger, anise, coriander, coffee, or a mint oil from any species of the genus Mentha), flavour enhancers, bitterness receptor site blockers, sensorial receptor site activators or stimulators, sugars and/or sugar substitutes (e.g., sucralose, acesulfame potassium, aspartame, saccharine, cyclamates, lactose, sucrose, glucose, fructose, sorbitol, or mannitol), and other additives such as charcoal, chlorophyll, minerals, botanicals, or breath freshening agents. They may be imitation, synthetic or natural ingredients or blends thereof. They may be in any suitable form, for example, oil, liquid, or powder.

The flavor may be added to the aerosolisable material as part of a so-called "flavour block", where one or more flavours are blended together and then added to the aerosolisable material.

In some embodiments, the olfactory active constituent comprises a terpene. In some embodiments, the terpene is a terpene derivable from a phytocannabinoid producing plant, such as a plant from the stain of the cannabis sativa species, such as hemp. In some embodiments, the aerosolisable material comprises a cannabinoid isolate in combination with a terpene derivable from a phytocannabinoid producing plant.

Suitable terpenes in this regard include so-called "C10" terpenes, which are those terpenes comprising 10 carbon atoms, and so-called "C15" terpenes, which are those terpenes comprising 15 carbon atoms.

In some embodiments, the aerosolisable material comprises more than one terpene. For example, the aerosolisable material may comprise one, two, three, four, five, six, seven, eight, nine, ten or more terpenes as defined herein.

In some embodiments, the terpene is selected based on its solubility in a propylene glycol/glycerol system.

In this regard, the terpene may be selected on the basis of being soluble when present in a propylene glycol/glycerol system, where the w/w% amount of propylene glycol C_{%} present in the material, based on the total weight of the material, is determined on the basis of the following relationship: ${C}_{%} = 11.416 \times {\left(T\right)}^{0.377}$ wherein T is the amount of the at least one terpene present in the material in mg/ml.

By ensuring the selected terpene meets the above threshold when present in a propylene glycol/glycerol system, the stability of the system will not be substantially compromised by including a terpene. In other words, the terpene(s) may be selected such that their solubility in propylene glycol is substantially matched to that of cannabidiol.

In some embodiments, the terpene is selected from pinene (alpha and beta), geraniol, linalool, limonene, carvone, eucalyptol, menthone, 1,8-cineole, iso-menthone, piperitone, myrcene, beta-bourbonene, germacrene and mixtures thereof.

In some embodiments, the aerosolisable material comprises a combination of terpenes. In some embodiments, the combination of terpenes may comprise a combination of at least geraniol and linalool. In some embodiments, the combination of terpenes may comprise a combination of at least eucalyptol and menthone. In some embodiments, the combination of terpenes may comprise a combination of at least eucalyptol, carvone, piperitone and menthone. In some embodiments, the combination of terpenes may comprise a combination of at least eucalyptol, carvone, beta-bourbonene, germacrene, piperitone, iso-menthone and menthone.

In one embodiment, the terpene(s) are present in a flavour block. This means that the terpenes are blended with one or more other flavours (optionally with an appropriate solvent, for example propylene glycol) and then the flavour block is added during the manufacture of the aerosolisable material. In some embodiments, the total amount of the flavour block present in the aerosolisable material is up to about 10 w/w%. In some embodiments, the total amount of the flavour block present in the aerosolisable material is up to about 9 w/w%. In some embodiments, the total amount of the flavour block present in the aerosolisable material is up to about 8 w/w%. In some embodiments, the total amount of the flavour block present in the aerosolisable material is up to about 7 w/w%. In some embodiments, the total amount of the flavour block present in the aerosolisable material is up to about 6 w/w%. In some embodiments, the total amount of the flavour block present in the aerosolisable material is up to about 5 w/w%.

In one embodiment, the total amount of terpene present in the aerosolisable material is up to about 10 mg/ml. In one embodiment, the total amount of terpene present in the aerosolisable material is up to about 9 mg/ml. In one embodiment, the total amount of terpene present in the aerosolisable material is up to about 8 mg/ml. In one embodiment, the total amount of terpene present in the aerosolisable material is up to about 7 mg/ml. In one embodiment, the total amount of terpene present in the aerosolisable material is up to about 6 mg/ml. In one embodiment, the total amount of terpene present in the aerosolisable material is up to about 5 mg/ml. In one embodiment, the total amount of terpene present in the aerosolisable material is up to about 4 mg/ml. In one embodiment, the total amount of terpene present in the aerosolisable material is up to about 3 mg/ml. In one embodiment, the total amount of terpene present in the aerosolisable material is up to about 2 mg/ml. In one embodiment, the total amount of terpene present in the aerosolisable material is up to about 1 mg/ml.

In one embodiment, the total amount of terpene present in the aerosolisable material is from about 0.1 mg/ml up to about 10 mg/ml. In one embodiment, the total amount of terpene present in the aerosolisable material is from about 0.2 mg/ml up to about 10 mg/ml. In one embodiment, the total amount of terpene present in the aerosolisable material is from about 0.3 mg/ml up to about 10 mg/ml. In one embodiment, the total amount of terpene present in the aerosolisable material is from about 0.4 mg/ml up to about 10 mg/ml. In one embodiment, the total amount of terpene present in the aerosolisable material is from about 0.5 mg/ml up to about 10 mg/ml. In one embodiment, the total amount of terpene present in the aerosolisable material is from about 1.0 mg/ml up to about 10 mg/ml. In one embodiment, the total amount of terpene present in the aerosolisable material is from about 2.0 mg/ml up to about 10 mg/ml. In one embodiment, the total amount of terpene present in the aerosolisable material is from about 3.0 mg/ml up to about 10 mg/ml. In one embodiment, the total amount of terpene present in the aerosolisable material is from about 4.0 mg/ml up to about 10 mg/ml. In one embodiment, the total amount of terpene present in the aerosolisable material is from about 5.0 mg/ml up to about 10 mg/ml.

In one embodiment, the total amount of terpene present in the aerosolisable material is from about 0.1 mg/ml up to about 9.0 mg/ml. In one embodiment, the total amount of terpene present in the aerosolisable material is from about 0.1 mg/ml up to about 8.0 mg/ml. In one embodiment, the total amount of terpene present in the aerosolisable material is from about 0.1 mg/ml up to about 7.0 mg/ml. In one embodiment, the total amount of terpene present in the aerosolisable material is from about 0.1 mg/ml up to about 6.0 mg/ml. In one embodiment, the total amount of terpene present in the aerosolisable material is from about 0.1 mg/ml up to about 5.0 mg/ml. In one embodiment, the total amount of terpene present in the aerosolisable material is from about 0.1 mg/ml up to about 1 mg/ml. In one embodiment, the total amount of terpene present in the aerosolisable material is from about 0.1 mg/ml up to about 0.9 mg/ml. In one embodiment, the total amount of terpene present in the aerosolisable material is from about 0.1 mg/ml up to about 0.8 mg/ml. In one embodiment, the total amount of terpene present in the aerosolisable material is from about 0.1 mg/ml up to about 0.7 mg/ml. In one embodiment, the total amount of terpene present in the aerosolisable material is from about 0.1 mg/ml up to about 0.6 mg/ml. In one embodiment, the total amount of terpene present in the aerosolisable material is from about 0.1 mg/ml up to about 0.5 mg/ml.

For the avoidance of doubt, combinations of the above end points are explicitly envisaged by the present disclosure. This applies to any of the ranges disclosed herein.

The one or more other functional constituents may comprise one or more of pH regulators, colouring agents, preservatives, binders, fillers, and/or antioxidants. In particular, the pH regulator may include one or more acids selected from organic or inorganic acids. An example of an inorganic acid is phosphoric acid. The organic acid may include a carboxylic acid. The carboxylic acid may be any suitable carboxylic acid. In one embodiment the acid is a mono-carboxylic acid. In one embodiment the acid may be selected from the group consisting of acetic acid, lactic acid, formic acid, citric acid, benzoic acid, pyruvic acid, levulinic acid, succinic acid, tartaric acid, oleic acid, sorbic acid, propionic acid, phenylacetic acid, and mixtures thereof.

In some embodiments, an acceptable turbidity is achieved without the use of any/significant amounts of water. In this regard, whilst water may otherwise assist in the preparation of aerosolisable materials since water containing materials may have a lower viscosity and therefore may be transferred more easily to an aerosol generating component, it has been found in the context of the present disclosure that water can negatively influence the stability of the aerosolisable material containing at least one cannabinoid.

In some embodiments, the aerosolisable material comprises less than 12%w/w water. In some embodiments, the aerosolisable material comprises less than 11%w/w water. In some embodiments, the aerosolisable material comprises less than 10%w/w water. In some embodiments, the aerosolisable material comprises less than 5%w/w water.

In some embodiments, the aerosolisable material comprises less than 1%w/w water.

In some embodiments, the aerosolisable material comprises less than 0.5%w/w water. In some embodiments, the aerosolisable material comprises substantially no water.

In particular, it has been found that for certain aerosolisable materials comprising a cannabinoid, such as cannabidiol, if the material comprises water in amounts of about 12%w/w, the cannabinoid is rendered unstable.

In a further aspect there is provided an article comprising the aerosolisable material as defined herein.

The article may be a container, such as a bottle, or may be a component for use with an aerosol provision device.

For example, the article may comprise an area (store) for receiving the aerosolisable material defined herein, an aerosol generating component, an aerosol generating area, and/or a mouthpiece.

In some embodiments, there is provided an article for use with an aerosol provision system, the article comprising a store comprising an aerosolisable material as defined herein, an aerosol generating component (such as a heater), an aerosol generating area, a transport element, and a mouthpiece.

Aerosolisable material may be transferred from the store for receiving an aerosolisable material to the aerosol generating component via a transport element, such as a wick, pump or the like. The skilled person is able to select suitable transport elements depending on the type of aerosolisable material that is to be transported and the rate at which it must be supplied.

Particular mention may be made of transport elements, such as wicks, formed from fibrous materials, foamed materials, sintered materials, woven and non-woven materials.

An airflow pathway typically extends through the article (optionally via the device) to an outlet. The pathway is oriented such that generated aerosol is entrained in the airflow such that it can be delivered to the outlet for inhalation by a user.

In one embodiment, the aerosol generating component is a heater.

Typically, the area for receiving an aerosolisable material will allow for the article to be refilled with aerosolisable material as the aerosolisable material is depleted during use.

Figure 1 is a highly schematic diagram (not to scale) of an example aerosol provision system, such as an e-cigarette 10, to which embodiments are applicable. The e-cigarette has a generally cylindrical shape, extending along a longitudinal axis indicated by a dashed line (although aspects of the invention are applicable to e-cigarettes configured in other shapes and arrangements), and comprises two main components, namely an aerosol provision device 20 and an article 30.

The article 30 includes a store for aerosolisable material (source liquid) 38 containing an aerosolisable material (source liquid) from which an aerosol is to be generated. The article 30 further comprises an aerosol generating component (heating element or heater) 36 for heating aerosolisable material to generate the aerosol. A transport element or wicking element or wick 37 is provided to deliver aerosolisable material from the store 38 to the heating element 36. A part or parts of the wick 37 are in fluid communication with aerosolisable material in the store 38 and by a wicking or capillary action aerosolisable material is drawn along or through the wick 37 to a part or parts of the wick 37 which are in contact with the heater 36.

Vaporization of the aerosolisable material occurs at the interface between the wick 37 and the heater 36 by the provision of heat energy to the aerosolisable material to cause evaporation, thus generating the aerosol. The aerosolisable material, the wick 37 and the heater 36 may be collectively referred to as an aerosol or vapour source. The wick 37 and the heater 36 may be collectively referred to as a vaporizer or an atomiser 15.

Typically a single wick will be present, but it is envisaged that more than one wick could be present, for example, two, three, four or five wicks.

As described above, the wick may be formed a sintered material. The sintered material may comprise sintered ceramic, sintered metal fibers/powders, or a combination of the two. The (or at least one of/all of the) sintered wick(s) may have deposited thereon/embedded therein an electrically resistive heater. Such a heater may be formed from heat conducting alloys such as NiCr alloys. Alternatively, the sintered material may have such electrical properties such that when a current is passed there through, it is heated. Thus, the aerosol generating component and the wick may be considered to be integrated. In some embodiments, the aerosol generating component and the wick are formed from the same material and form a single component.

In some embodiments, the wick is formed from a sintered metal material and is generally in the form of a planar sheet. Thus, the wick element may have a substantially thin flat shape. For example it may be considered as a sheet, layer, film, substrate or the like. By this it is meant that a thickness of the wick is less or very much less than at least one of the length and the width of the wick. Thus, the wick thickness (its smallest dimension) is less or very much less than the longest dimension.

The wick may be made of a homogenous, granular, fibrous or flocculent sintered metal(s) so as to form said capillary structure. Wick elements can be made from a conductive material which is a nonwoven sintered porous web structure comprising metal fibres, such as fibres of stainless steel. For example, the stainless steel may be AISI (American Iron and Steel Institute) 316L (corresponding to European standard 1.4404). The material's weight may be in the range of 100 - 300 g/m².

Where the wick is generally planar, the thickness of the wick may be in the range of 75 - 250 µm. A typical fibre diameter may be about 12 µm, and a typical mean pore size (size of the voids between the fibres) may be about 32 µm. An example of a material of this type is Bekipor (RTM) ST porous metal fibre media manufactured by NV Bekaert SA, Belgium, being a range of porous nonwoven fibre matrix materials made by sintering stainless steel fibres.

Note also that while the material is described as planar, this refers to the relative dimensions of the sheet material and the wick (a thickness many times smaller than the length and/or width) but does not necessarily indicate flatness, in particular of the final wick made from the material. A wick may be flat but might alternatively be formed from sheet material into a non-flat shape such as curved, rippled, corrugated, ridged, formed into a tube or otherwise made concave and/or convex.

The wick element may have various properties. It is formed from a porous material to enable the required wicking or capillary effect for drawing source liquid through it from an store for aerosolisable material (where the wick meets the aerosolisable material at a store contact site) to the vaporisation interface. Porosity is typically provided by a plurality of interconnected or partially interconnected pores (holes or interstices) throughout the material, and open to the outer surface of the material. Any level of porosity may be employed depending on the material, the size of the pores and the required rate of wicking. For example a porosity of between 30% and 85% might be selected, such as between 40% and 70%, between 50% and 80%, between 35% and 75% or between 40% and 75%. This might be an average porosity value for the whole wick element, since porosity may or may not be uniform across the wick. For example, pore size at the store contact site might be different from pore size nearer to the heater.

It is useful for the wick to have sufficient rigidity to support itself in a required within the article. For example, it may be mounted at or near one or two edges and be required to maintain its position substantially without flexing, bending or sagging.

As an example, porous sintered ceramic is a useful material to use as the wick element. Any ceramic with appropriate porosity may be used. If porous ceramic is chosen as the porous wick material, this is available as a powder which can be formed into a solid by sintering (heating to cause coalescence, possibly under applied pressure). Sintering then solidifies the ceramic to create the porous wick.

The article 30 further includes a mouthpiece 35 having an opening through which a user may inhale the aerosol generated by the vaporizer 15. The aerosol for inhalation may be described as an aerosol stream or inhalable airstream.

The aerosol delivery device 20 includes a power source (a re-chargeable cell or battery 14, referred to herein after as a battery) to provide power for the e-cigarette 10, and a controller (printed circuit board (PCB)) 28 and/or other electronics for generally controlling the e-cigarette 10. The aerosol delivery device can therefore also be considered as a battery section, or a control unit or section.

During operation of the device, the controller will determine that a user has initiated a request for the generation of an aerosol. This could be done via a button on the device which sends a signal to the controller that the aerosol generator should be powered. Alternatively, a sensor located in or proximal to the airflow pathway could detect airflow through the airflow pathway and convey this detection to the controller. A sensor may also be present in addition to the presence of a button, as the sensor may be used to determine certain usage characteristics, such as airflow, timing of aerosol generation etc.

For example, in use, when the heater 36 receives power from the battery 14, as controlled by the circuit board 28 possibly in response to pressure changes detected by an air pressure sensor (not shown), the heater 36 vaporizes aerosolisable material delivered by the wick 37 to generate the aerosol, and this aerosol stream is then inhaled by a user through the opening in the mouthpiece 35. The aerosol is carried from the aerosol source to the mouthpiece 35 along an air channel (not shown in Figure 1) that connects the aerosol source to the mouthpiece opening as a user inhales on the mouthpiece.

In this particular example, the device 20 and article 30 are detachable from one another by separation in a direction parallel to the longitudinal axis, as shown in Figure 1, but are joined together when the system 10 is in use by cooperating engagement elements 21, 31 (for example, a screw, magnetic or bayonet fitting) to provide mechanical and electrical connectivity between the device 20 and the article 30, in particular connecting the heater 36 to the battery 14. The battery may be charged as is known to one skilled in the art.

In some embodiments, the article comprises/forms a sealed container. For example, the sealed container may be hermetically sealed. The present inventors have found that inclusion of the aerosolisable material in a sealed article assists in preventing water ingress into the system, which can prevent the cannabidiol from precipitating. The hermetically sealed container may comprise a blister pack with one or more hermetically sealed compartments for storage of one or more articles comprising the aerosolisable material described herein.

In some embodiments, the article comprises a housing within which the aerosolisable material is contained. The housing may be transparent such that the aerosolisable material can be viewed from outside of the housing. It may also be that the housing has a degree of opacity such that the passage of light through the housing is limited. This can be important so as to prevent light (such as ultra violet light) from entering the housing and compromising the stability of the aerosolisable material. In this regard, the present inventors have considered that cannabinoids may be particularly susceptible to such light destabilization. In some embodiments, the housing is formed from a material which inhibits the passage of ultra violet light there through. In some embodiments, it may be that the sealed container mentioned above is formed from a material which has a degree of opacity such that the passage of light through the sealed container is limited. Further, the sealed container mentioned above may be formed from a material which inhibits/prevents the passage of ultra violet light there through. This may be in addition to said sealed container being hermetically sealed and/or comprising a blister pack with one or more hermetically sealed compartments for storage of one or more articles comprising the aerosolisable material described herein.

In a further aspect there is provided an aerosol provision system comprising an aerosol provision device and an article as defined herein.

In a further aspect there is provided a method for producing the aerosolisable material as defined herein, the method comprising combining (+)-cannabidiol with at least one carrier constituent.

In this regard, the present method may be further defined according to any of the features described above.

In some embodiments, the method further excludes a step of adding water and/or a surface active agent to the aerosolisable material.

In a further aspect, there is provided a method for producing an aerosol comprising generating an aerosol from an aerosolisable material as defined herein.

The various embodiments described herein are presented only to assist in understanding and teaching the claimed features. These embodiments are provided as a representative sample of embodiments only, and are not exhaustive and/or exclusive. It is to be understood that advantages, embodiments, examples, functions, features, structures, and/or other aspects described herein are not to be considered limitations on the scope of the invention as defined by the claims or limitations on equivalents to the claims, and that other embodiments may be utilised and modifications may be made without departing from the scope of the claimed invention. Various embodiments of the invention may suitably comprise, consist of, or consist essentially of, appropriate combinations of the disclosed elements, components, features, parts, steps, means, etc., other than those specifically described herein.

## Claims

1. An aerosolisable material comprising (+)-cannabidiol and at least one carrier constituent.

2. The aerosolisable material according to claim 1, wherein at least 1%, preferably at least 50%, preferably at least 60%, preferably at least 70%, preferably at least 75%, preferably at least 80%, preferably at least 85%, preferably at least 90%, preferably substantially all, of the cannabidiol present in the aerosolisable material, is present in the form of (+)-cannabidiol.

3. The aerosolisable material according to any one of the preceding claims, wherein the carrier constituent comprises one or more of glycerol, propylene glycol, triethylene glycol, tetraethylene glycol, 1,3-butylene glycol, erythritol, meso-Erythritol, ethyl vanillate, ethyl laurate, a diethyl suberate, triethyl citrate, triethylene glycol diacetate, triacetin, a diacetin mixture, benzyl benzoate, benzyl phenyl acetate, tributyrin, lauryl acetate, lauric acid, myristic acid, and propylene carbonate.

4. The aerosolisable material according to claim 3, wherein the carrier constituent comprises propylene glycol; preferably wherein propylene glycol is present in an amount of at least 50%w/w based on the total weight of the material; preferably wherein propylene glycol is present in an amount of at least 60%w/w based on the total weight of the material; preferably wherein propylene glycol is present in an amount of at least 70%w/w based on the total weight of the material.

5. The aerosolisable material according to claim 3, wherein the carrier constituent comprises glycerol; preferably wherein glycerol is present in an amount of at least 50%w/w based on the total weight of the material; preferably wherein glycerol is present in an amount of at least 60%w/w based on the total weight of the material; preferably wherein glycerol is present in an amount of at least 70%w/w based on the total weight of the material.

6. The aerosolisable material according to claims 3 and 4, wherein both glycerol and propylene glycol are present as carrier constituents; preferably wherein, based on the total amount of propylene glycol and glycerol in the aerosolisable material, the aerosolisable material comprises:
60 to 90%w/w propylene glycol; and
40 to 10%w/w glycerol; preferably wherein, based on the total amount of propylene glycol and glycerol in the aerosolisable material, the aerosolisable material comprises:
70 to 80%w/w propylene glycol; and
30 to 20%w/w glycerol; wherein, based on the total amount of propylene glycol and glycerol in the aerosolisable material, the aerosolisable material comprises about 70%w/w propylene glycol and about 30% glycerol.

7. The aerosolisable material according to any one of the preceding claims, wherein the (+)-cannabinoid is present in an amount of 5mg/ml of material or more; wherein the (+)-cannabidiol is present in the material in an amount of 10mg/ml of material or more; preferably wherein the (+)-cannabidiol is present in the material in an amount of 30mg/ml of material or more; preferably wherein the (+)-cannabidiol is present in the material in an amount of 60mg/ml of material or more.

8. The aerosolisable material according to any one of the preceding claims, wherein the turbidity of the material is about 10 NTU or less.

9. The aerosolisable material according to claim 8, wherein the material takes the form of a liquid at about 25°C.

10. The aerosolisable material according to any one of the preceding claims, wherein the material comprises less than 12%w/w water.

11. The aerosolisable material according to any one of the preceding claims, wherein the material further comprises one or more active constituents in addition to the cannabidiol; preferably wherein the one or more active constituents is an olfactory active constituent.

12. An article comprising the aerosolisable material as defined in any one of claims 1 to 11; the article comprises an area (store) for receiving the aerosolisable material of any one of claims 1 to 11, an aerosol generating component, an aerosol generating area, a transport element and a mouthpiece; preferably wherein the aerosol generating component comprises a heater; preferably wherein the transport element is a wick.

13. An aerosol provision system comprising an aerosol provision device and an article as defined in claim 12.

14. A method for producing the aerosolisable material as defined in any one of claims 1 to 12, the method comprising combining (+)-cannabidiol with at least one carrier constituent; preferably wherein the method is characterized according to any one of claims 2 to 11.

15. A sealed container comprising the article according to claim 12; preferably wherein the container is hermetically sealed and is formed from a material which inhibits/prevents the passage of ultra violet light there through; preferably the sealed container comprising a blister pack with one or more hermetically sealed compartments.

## Patentansprüche

1. Aerosolierbares Material, umfassend (+)-Cannabidiol und mindestens einen Trägerbestandteil.

2. Aerosolierbares Material nach Anspruch 1, wobei mindestens 1 %, bevorzugt mindestens 50 %, bevorzugt mindestens 60 %, bevorzugt mindestens 70 %, bevorzugt mindestens 75 %, bevorzugt mindestens 80 %, bevorzugt mindestens 85 %, bevorzugt mindestens 90 %, bevorzugt im Wesentlichen das gesamte in dem aerosolierbaren Material vorhandene Cannabidiol in Form von (+)-Cannabidiol vorliegt.

3. Aerosolierbares Material nach einem der vorhergehenden Ansprüche, wobei der Trägerbestandteil Glycerin, Propylenglykol, Triethylenglykol, Tetraethylenglykol, 1,3-Butylenglykol, Erythrit, mesoErythrit, Ethylvanillat, Ethyllaurat, ein Diethylsuberat, Triethylcitrat, Triethylenglykoldiacetat, Triacetin, eine Diacetinmischung, Benzylbenzoat, Benzylphenylacetat, Tributyrin, Laurylacetat, Laurinsäure, Myristinsäure und/oder Propylencarbonat umfasst.

4. Aerosolierbares Material nach Anspruch 3, wobei der Trägerbestandteil Propylenglykol umfasst, wobei Propylenglykol vorzugsweise in einer Menge von mindestens 50 Gew.-%, bezogen auf das Gesamtgewicht des Materials, vorliegt, wobei Propylenglykol vorzugsweise in einer Menge von mindestens 60 Gew.-%, bezogen auf das Gesamtgewicht des Materials, vorliegt, wobei Propylenglykol vorzugsweise in einer Menge von mindestens 70 Gew.-%, bezogen auf das Gesamtgewicht des Materials, vorliegt.

5. Aerosolierbares Material nach Anspruch 3, wobei der Trägerbestandteil Glycerin umfasst, wobei Glycerin vorzugsweise in einer Menge von mindestens 50 Gew.-%, bezogen auf das Gesamtgewicht des Materials, vorliegt, wobei Glycerin vorzugsweise in einer Menge von mindestens 60 Gew.-%, bezogen auf das Gesamtgewicht des Materials, vorliegt, wobei Glycerin vorzugsweise in einer Menge von mindestens 70 Gew.-%, bezogen auf das Gesamtgewicht des Materials, vorliegt.

6. Aerosolierbares Material nach Anspruch 3 oder 4, wobei sowohl Glycerin als auch Propylenglykol als Trägerbestandteile vorliegen, wobei das aerosolierbare Material vorzugsweise, bezogen auf die Gesamtmenge an Propylenglykol und Glycerin in dem aerosolierbaren Material, Folgendes umfasst:
60 bis 90 Gew.-% Propylenglykol und
40 bis 10 Gew.-% Glycerin, wobei vorzugsweise, bezogen auf die Gesamtmenge an Propylenglykol und Glycerin in dem aerosolierbaren Material, das aerosolierbare Material Folgendes umfasst:
70 bis 80 Gew.-% Propylenglykol und
30 bis 20 Gew.-% Glycerin, wobei das aerosolierbare Material, bezogen auf die Gesamtmenge an Propylenglykol und Glycerin in dem aerosolierbaren Material, etwa 70 Gew.-% Propylenglykol und etwa 30 Gew.-% Glycerin umfasst.

7. Aerosolierbares Material nach einem der vorhergehenden Ansprüche, wobei das (+)-Cannabinoid in einer Menge von 5 mg/ml Material oder mehr vorhanden ist, wobei das (+)-Cannabidiol in dem Material in einer Menge von 10 mg/ml Material oder mehr vorhanden ist, wobei das (+)-Cannabidiol in dem Material vorzugsweise in einer Menge von 30 mg/ml Material oder mehr vorhanden ist, wobei das (+)-Cannabidiol in dem Material vorzugsweise in einer Menge von 60 mg/ml Material oder mehr vorhanden ist.

8. Aerosolierbares Material nach einem der vorhergehenden Ansprüche, wobei die Trübung des Materials etwa 10 NTU oder weniger beträgt.

9. Aerosolierbares Material nach Anspruch 8, wobei das Material bei etwa 25 °C die Form einer Flüssigkeit annimmt.

10. Aerosolierbares Material nach einem der vorhergehenden Ansprüche, wobei das Material weniger als 12 Gew.-% Wasser umfasst.

11. Aerosolierbares Material nach einem der vorhergehenden Ansprüche, wobei das Material weiterhin zusätzlich zu dem Cannabidiol einen oder mehrere aktive Bestandteile umfasst, wobei der eine oder die mehreren aktiven Bestandteile vorzugsweise ein olfaktorischer aktiver Bestandteil sind.

12. Artikel, der das aerosolierbare Material nach einem der Ansprüche 1 bis 11 umfasst, wobei der Artikel einen Bereich (Speicher) zur Aufnahme des aerosolierbaren Materials nach einem der Ansprüche 1 bis 11, eine aerosolerzeugende Komponente, einen aerosolerzeugenden Bereich, ein Transportelement und ein Mundstück umfasst; wobei die aerosolerzeugende Komponente vorzugsweise ein Heizelement umfasst, wobei das Transportelement vorzugsweise ein Docht ist.

13. Aerosolbereitstellungssystem, das eine Aerosolbereitstellungsvorrichtung und einen wie in Anspruch 12 definierten Artikel umfasst.

14. Verfahren zur Herstellung des aerosolierbaren Materials nach einem der Ansprüche 1 bis 12, wobei das Verfahren das Kombinieren von (+)-Cannabidiol mit mindestens einem Trägerbestandteil umfasst, wobei das Verfahren vorzugsweise nach einem der Ansprüche 2 bis **11** gekennzeichnet ist.

15. Verschlossener Behälter, umfassend den Artikel nach Anspruch 12, wobei der Behälter vorzugsweise hermetisch verschlossen ist und aus einem Material gebildet ist, das den Durchgang von ultraviolettem Licht durch den Behälter hemmt/verhindert, wobei der verschlossene Behälter vorzugsweise eine Blisterverpackung mit einem oder mehreren hermetisch verschlossenen Fächern umfasst.

## Revendications

1. Matériau aérosolisable comprenant du (+)-cannabidiol et au moins un constituant porteur.

2. Matériau aérosolisable selon la revendication 1, dans lequel au moins 1 %, de préférence au moins 50 %, de préférence au moins 60 %, de préférence au moins 70 %, de préférence au moins 75 %, de préférence au moins 80 %, de préférence au moins 85 %, de préférence au moins 90 %, de préférence sensiblement tout, du/le cannabidiol présent dans le matériau aérosolisable, est présent sous la forme de (+)-cannabidiol.

3. Matériau aérosolisable selon l'une quelconque des revendications précédentes, dans lequel le constituant porteur comprend un ou plusieurs parmi le glycérol, le propylène glycol, le triéthylène glycol, le tétraéthylène glycol, le 1,3-butylène glycol, l'érythritol, le méso-érythritol, le vanillate d'éthyle, le laurate d'éthyle, un subérate de diéthyle, le citrate de triéthyle, le diacétate de triéthylène glycol, la triacétine, un mélange de diacétine, le benzoate de benzyle, l'acétate de benzyle et de phényle, la tributyrine, l'acétate de lauryle, l'acide laurique, l'acide myristique et le carbonate de propylène.

4. Matériau aérosolisable selon la revendication 3, dans lequel le constituant porteur comprend du propylène glycol ; de préférence dans lequel le propylène glycol est présent en une quantité d'au moins 50 % p/p sur la base du poids total du matériau ; de préférence dans lequel le propylène glycol est présent en une quantité d'au moins 60 % p/p sur la base du poids total du matériau ; de préférence dans lequel le propylène glycol est présent en une quantité d'au moins 70 % p/p sur la base du poids total du matériau.

5. Matériau aérosolisable selon la revendication 3, dans lequel le constituant porteur comprend du glycérol ; de préférence dans lequel le glycérol est présent en une quantité d'au moins 50 % p/p sur la base du poids total du matériau ; de préférence dans lequel le glycérol est présent en une quantité d'au moins 60 % p/p sur la base du poids total du matériau ; de préférence dans lequel le glycérol est présent en une quantité d'au moins 70 % p/p sur la base du poids total du matériau.

6. Matériau aérosolisable selon les revendications 3 et 4, dans lequel le glycérol et le propylène glycol sont tous deux présents en tant que constituants porteurs ; de préférence dans lequel, sur la base de la quantité totale de propylène glycol et de glycérol dans le matériau aérosolisable, le matériau aérosolisable comprend :
60 à 90 % p/p de propylène glycol ; et
40 à 10 % p/p de glycérol ; préférablement dans lequel, sur la base de la quantité totale de propylène glycol et de glycérol dans le matériau aérosolisable, le matériau aérosolisable comprend :
70 à 80 % p/p de propylène glycol ; et
30 à 20 % p/p de glycérol ; dans lequel, sur la base de la quantité totale de propylène glycol et de glycérol dans le matériau aérosolisable, le matériau aérosolisable comprend environ 70 % p/p de propylène glycol et environ 30 % de glycérol.

7. Matériau aérosolisable selon l'une quelconque des revendications précédentes, dans lequel le (+)-cannabinoïde est présent en une quantité de 5 mg/ml de matériau ou plus ; dans lequel le (+)-cannabidiol est présent dans le matériau en une quantité de 10 mg/ml de matériau ou plus ; de préférence dans lequel le (+)-cannabidiol est présent dans le matériau en une quantité de 30 mg/ml de matériau ou plus ; de préférence dans lequel le (+)-cannabidiol est présent dans le matériau en une quantité de 60 mg/ml de matériau ou plus.

8. Matériau aérosolisable selon l'une quelconque des revendications précédentes, dans lequel la turbidité du matériau est d'environ 10 UTN ou moins.

9. Matériau aérosolisable selon la revendication 8, dans lequel le matériau prend la forme d'un liquide à environ 25 °C.

10. Matériau aérosolisable selon l'une quelconque des revendications précédentes, dans lequel le matériau comprend moins de 12 % p/p d'eau.

11. Matériau aérosolisable selon l'une quelconque des revendications précédentes, dans lequel le matériau comprend en outre un ou plusieurs constituants actifs en plus du cannabidiol ; de préférence dans lequel le ou les constituants actifs sont un constituant actif olfactif.

12. Article comprenant le matériau aérosolisable tel que défini dans l'une quelconque des revendications 1 à 11 ; l'article comprenant une zone (réservoir) pour recevoir le matériau aérosolisable selon l'une quelconque des revendications 1 à 11, un composant générateur d'aérosol, une zone de génération d'aérosol, un élément de transport et un embout buccal ; de préférence dans lequel le composant de génération d'aérosol comprend un élément chauffant ; de préférence dans lequel l'élément de transport est une mèche.

13. Système de fourniture d'aérosol comprenant un dispositif de fourniture d'aérosol et un article tel que défini dans la revendication 12.

14. Procédé de production du matériau aérosolisable tel que défini dans l'une quelconque des revendications 1 à 12, le procédé comprenant la combinaison de (+)-cannabidiol avec au moins un constituant porteur ; de préférence, dans lequel le procédé est caractérisé selon l'une quelconque des revendications 2 à 11.

15. Récipient scellé comprenant l'article selon la revendication 12 ; de préférence dans lequel le récipient est scellé hermétiquement et est formé à partir d'un matériau qui inhibe/empêche le passage de lumière ultraviolette à travers celui-ci ; de préférence le récipient scellé comprenant un emballage blister comportant un ou plusieurs compartiments scellés hermétiquement.
